# EUROPEAN PATENT APPLICATION

(11) **EP 1 192 886 A1**
(43) Date of publication of application: **03.04.2002**
(21) Application number: 00121590.4
(22) Date of filing: 02.10.2000
(51) Int. Cl.: A47G 9/02, A47C 21/06, A61L 15/46

(54) **Disposable, moisture vapour permeable, liquid impermeable covering sheet for bedding articles having odour reduction properties**

(71) Applicant: The Procter & Gamble Company, Cincinnati, Ohio 45202 (US)
(72) Inventor: Porter, Philip, 1150 Brussels (BE); Russo, Elisabetta, 00142 Rome (IT); Sirianni, Giuseppe, 00165 Rome (IT); Trujillo, Rafael, 1410 Waterloo (BE); Pesce, Antonella, 65120 Pescara (IT); Bonfanti, Lidia, 66020 Sambuceto di S. Giovanni Chietino (IT)
(74) Representative: Veronese, Pancrazio

(57) **Abstract**

The present invention relates to a disposable covering sheet which is moisture vapour permeable and liquid impermeable and has odour control properties and, if desired, also active agent releasing properties. The disposable covering sheet comprises at least a moisture vapour permeable layer and a liquid impermeable but moisture vapour permeable film layer of thermoplastic compositions. The disposable covering sheet such as, for example, a mattress cover or a pillow cover gives a better comfort to the user by providing odour control properties and, if desired, also active agent releasing properties. The disposable covering sheet of the present invention can find a variety of applications wherein moisture vapour permeability and liquid imperviousness combined with specific odour control properties and preferably also active agent releasing properties are desirable.

## Description

### Field of the Invention

The present invention relates to a disposable covering sheet preferably for bedding items such as mattresses, pillows, etc. which is moisture vapour permeable, liquid impermeable, and has odour reduction properties. The covering sheet comprises liquid impermeable formed structures having a moisture vapour permeability comprising thermoplastic compositions preferably applied onto a moisture vapour permeable substrate. The covering sheet of the present invention can find a variety of applications wherein moisture vapour permeability, liquid imperviousness, disposability, comfort, specific odour control properties and, if desired, also active agents releasing properties are desirable. Preferably the covering sheet of the instant invention is a mattress cover or a pillow cover.

### Background of the Invention

Covering sheets, particular covering sheets for bedding articles, such as bed mattress covers, are known in the art. A primary use of these articles is to protect mattresses, particularly bed mattresses, but also pillows, cushions, duvets, upholstery, from contaminants, e.g. from dust, liquids, or bodily fluids, for example in environments where a mattress is intended to be used by different users, such as in hospitals, hotels, or rental houses. Furthermore, mattress covers are used in house for providing a mattress with a protective covering.

More recently mattress covers have been also proposed to act as an allergen barrier to control house mites that live in the dust, typically on and within mattresses where they can find favourable environmental conditions in terms of temperature and nourishment.

It has long been established that house mites, also known as dust mites, are a source of house dust allergens that not only cause allergies, but also adversely contribute to other pathologies, such as asthma. It has also been established that use of allergen control measures is effective in controlling these conditions. Allergen-proof encasing to contain mites to prevent allergen egress has long been used in mattresses, such as bed mattresses, pillows, cushions, duvets, upholstery.

Mattress covers come in direct or indirect (i.e. through further intermediate layers, e.g. bed sheets, or pillowcases) contact with the human body, therefore it is important that such covers are moisture vapour permeable for comfort reasons, in addition to being liquid impermeable in order to provide the mattress with the desired protection against external agents. When used as a barrier against dust mites they should also have an allergen barrier capability.

Various examples of disposable mattress covers which are at the same time liquid impermeable and breathable, i.e. moisture vapour permeable, are known in the art.

Known disposable mattress covers comprise different materials or structures which are capable of providing a liquid barrier, in addition to providing moisture vapour permeability, preferably air permeability. Such structures or materials can comprise a single layer, or multiple layers laminated together. An example are structures comprising thermoplastic microporous films, e.g. laminated to fibrous layers such as nonwoven layers.

Particularly preferred materials which are suitable for disposable mattress covers are hydrophilic continuous films, also known as "monolithic films", that do not allow the flow of moisture vapour through open pores or apertures in the material, but do transfer substantial amounts of moisture vapour through the film by absorbing water on one side of the film where the moisture vapour concentration is higher, and desorbing or evaporating it on the opposite side of the film where the moisture vapour concentration is lower.

In our patent applications EP 0963760 and EP 0964026 thermoplastic compositions are disclosed for making hydrophilic continuous moisture vapour permeable, liquid impermeable layers having preferred characteristics of moisture vapour permeability and liquid imperviousness. The disclosed preferred thermoplastic compositions are also readily processable so as to provide a coating having the desired thickness onto a substrate, so avoiding the need of complex traditional extrusion apparatuses. This is achieved by modifying the viscosity of the thermoplastic polymers by means of the inclusion in the composition of a suitable plasticiser or blend of plasticisers that lowers such viscosity. This allows to utilise with these preferred compositions typical process conditions known in the art for the direct coating of low viscosity hot melt compositions onto a substrate in order to form a moisture vapour permeable, liquid impervious film or layer.

Disposable, moisture vapour permeable, liquid impermeable mattress covers comprising a moisture vapour permeable, liquid impermeable structure, which in turn comprises a layer of the thermoplastic compositions according to either EP 0963760 or EP 0964026, preferably applied onto a moisture vapour permeable substrate, e.g. a fibrous layer such as a nonwoven layer in order to form a composite structure, are particularly preferred since they provide better moisture vapour permeability combined with liquid imperviousness, and also take advantage of the increased ease of manufacture associated to the above mentioned compositions.

Another improvement to disposable mattress covers is described in our European patent application No. 99124535.8, filed on 9 December 1999, where an increased friction is established between the disposable mattress cover and the mattress surface during the use, when i.e. the mattress cover is typically subjected to a certain compression against the mattress itself, in order to minimise possible misplacements of the cover during its use, for example when the mattress is a bed mattress or a pillow, and the mattress cover can be moved or misplaced by the user's movement, e.g. during the sleep. This is particularly important when the mattress cover is intended for partial covering of the external surface of a mattress, for example of the upper surface of a bed mattress, e.g., in combination with traditional means for connecting the cover to the mattress, such as for example elastic bands or strands applied at the corners of a rectangular bed mattress cover.

Provision of an increased friction between the mattress cover and the mattress is however also useful in the context of a mattress cover intended for total encasing of a mattress. In both cases in fact this increased friction prevents or at least reduces relative movements between the mattress cover and the mattress during the use, therefore helping in keeping the mattress cover in its right position provided initially, e.g. by the user when applying the cover to the mattress.

Even if the covering sheets disclosed in our above mentioned patent applications function well, particularly in terms of protection, they can be improved in terms of further benefits for the user by providing them with odour reduction properties, and also preferably with active agents releasing properties. While systems for releasing active agents such as perfumes, etc. are known in the art and also in the field of covering sheets for bedding articles, see for example WO 99/17643, and while odour control means are known in the art and particularly in the field of absorbent articles such as sanitary napkins, disposable diapers, incontinence products, etc., it is not known a disposable covering sheet, particularly for bedding items such as mattresses, pillows, etc. which is moisture vapour permeable, liquid impermeable and has specific odour controlling properties, preferably combined with active agents releasing properties.

### Summary of the Invention

A disposable, moisture vapour permeable, liquid impermeable, covering sheet comprising at least a moisture vapour permeable layer and a liquid impermeable but moisture vapour permeable film layer preferably applied onto said moisture vapour permeable layer, the covering sheet has specific odour controlling properties and, if desired, also active agent releasing properties.

More specifically said covering sheet comprises odour control means which are able to provide a malodour reduction of at least 15% as indicated hereinafter, as measured according to the Odour Control Test described herein.

### Brief Description of the Drawings

The drawings herein are intended to illustrate preferred embodiments of the present invention, but are not meant to limit in any way its scope.
Figure 1 is a schematic plan view of a disposable mattress cover according to the present invention.
Figure 2 is a cross-sectional view of the disposable mattress cover of figure 1 taken along the line A-A.
Figure 3 illustrates the mattress cover applied to a mattress.

### Detailed Description of the Invention

According to the present invention, a disposable, moisture vapour permeable, liquid impermeable covering sheet is provided which comprises at least a moisture vapour permeable layer and a liquid impermeable but moisture vapour permeable film layer comprising a highly processable thermoplastic composition having moisture vapour permeability. Said covering sheet offers a further benefit to the user, particularly when used as a covering sheet for bedding articles by providing odour control properties and, if desired, also active agent releasing properties.

Covering sheets according to the present invention can be used to cover and protect different articles in a variety of fields for both domestic, industrial or institutional usage; such usages include, but are not limited to, covering sheets for headrests and seats, both for domestic use or institutional use, and for use in the transportation industry, table covers of dining sets, covering sheets for medical examination tables, covering sheets for furniture, etc.

The preferred utilization of the covering sheets herein is in bedding articles, both for domestic and institutional usage. The covering sheets of the present invention are particularly useful for use in disposable bed mattress covers and pillow cases or covers.

The term "mattress", as used therein, refers to a fabric case filled with resilient material, such as for example cotton, hair, feathers, foam rubber, or an arrangement of coil springs, and therefore comprises particularly bed mattresses, but also pillows, cushions, comforters, duvets, upholstered portions of beds (such as headboards), or of sofas or armchairs. Consequently, even if the present invention will be described with particular reference to a disposable covering sheet for bedding items in the form of a mattress cover, it is not meant to limit in any way its scope to mattress covers.

Referring particularly to mattresses it is generally known that each mattress every year "absorbs" from about 45 kilos to about 80 kilos of sweat and about 150 grams of skin scales. In addition to this, millions of mites generally live in the mattress. Another problem is that accidental staining is practically unavoidable. Usually consumers are aware of the above problems and, in fact, most of them use reusable mattress covers which usually are made from cotton. But these covers do not solve the above problems and, in particular, they are not capable of providing an effective action of odour reduction, both towards odours already present in the mattress, and which can be released in the environment and smelled by the user, and odours, typically body odours, which instead can be transmitted to the mattress, for example by means of bodily fluids, typically sweat, as explained above, absorbed by the mattress during the use.

The disposable covering sheet of the present invention consists in a laminate of different layers comprising at least a first moisture vapour permeable layer and a second liquid impermeable but moisture vapour permeable film layer of thermoplastic composition bonded onto said first layer, i.e. the laminate preferably consists of at least two bonded layers giving rise to a composite structure.

If more film layers of thermoplastic compositions are utilised in a composite structure in combination with one or more other moisture vapour permeable layers to create a layered moisture vapour permeable, liquid impermeable composite structure, each film layer can be made of a same specific thermoplastic composition, or of different specific thermoplastic compositions.

The above composite structure can involve at least one component, typically the film layer, of the thermoplastic composition in combination with one or more other moisture vapour permeable layers. Such moisture vapour permeable materials include, but are not limited to: fibres, fibrous batts, non-wovens, wovens, papers, micro-porous or porous membranes, films such as polymeric films, perforated or apertured films and papers, macroscopically expanded films, cloth, etc.

Said other components may be non-absorbent, absorbent, liquid-containing, etc.

The composite structures of the covering sheets described above have a moisture vapour transfer rate (WVTR) of at least 400 g/m²•24h, more preferably at least 500 g/m²·24h, and most preferably at least 600 g/m²•24h, but they are impermeable to liquids.

According to a particularly preferred embodiment of the present invention the disposable, moisture vapour permeable, liquid impermeable mattress cover comprises a composite structure comprising a film layer of the thermoplastic composition applied onto a moisture vapour permeable substrate, wherein said film layer is intended, in use, to directly contact the mattress. The moisture vapour permeable substrate preferably comprises a fibrous layer, e.g. a nonwoven layer onto which the thermoplastic composition is applied in a continuous layer or film by means of known means, e.g. preferably by hot melt coating.

A disposable, moisture vapour permeable, liquid impermeable mattress cover comprising a moisture vapour permeable, liquid impermeable structure in turn comprising a layer of the thermoplastic composition which is intended, in use, to directly contact a mattress, can have any shape and arrangement which is known in the art, and which is suitable for its intended use.

For example, if the mattress consists of a standard bed mattress, the mattress cover can be a substantially rectangular flat sheet with elastic strings at the corners, or alternatively with integrally elasticised corners, as described in FR 2747899. The rectangular sheet typically has suitable dimensions in order to completely cover at least the upper surface of the bed mattress, and preferably, as in the latter embodiment referred to above, also the side surfaces thereof, wherein the elastic strings or the elasticised corners provide for a suitable releasable connection, in a way known in the art, to the mattress. Alternative configurations where the mattress cover has a suitable shape and size in order to completely encase a mattress are also possible. This latter configuration could be preferred when the mattress cover, in addition to liquid imperviousness and moisture vapour permeability, should also provide a total barrier against dust mites and related allergens.

Preferably, as shown in the herein drawings, a disposable mattress cover comprises a rectangular blanket made according to the present invention. In particular, the disposable, moisture vapour permeable, liquid impermeable blanket is formed by a topsheet 1, which is preferably a moisture vapour permeable fibrous layer, and a backsheet 2, which is a liquid impermeable but moisture vapour permeable film layer of a thermoplastic composition laminated onto said topsheet 1 in order to form a two-layer composite structure. The backsheet 2 is intended to face and to be in direct contact with the top of a mattress 4, while the topsheet 1 is intended to face the user.

The thermoplastic compositions can provide a certain degree of residual tackiness to the backsheet 2. In turn, this tackiness prevents or at least reduces relative movements between the mattress cover and the mattress 4 during use, therefore helping in keeping the mattress cover in its right position provided by the user initially. In any case and if preferred, the mattress cover can have no residual tackiness by suitably selecting the thermoplastic compositions or by neutralising any residual tackiness with known means, e.g. talcum powder. This tackiness can be also useful for incorporating the odour control materials and the active agents in the mattress cover according to the present invention.

For assisting the user to put the mattress cover on the mattress in the right position but also for fixing the mattress cover to the mattress in a stable manner adhesive means 3 are provided, as shown in the drawings. The adhesive means 3 can be one or more stripes of a conventional pressure sensitive adhesive covered by a release liner for protecting the adhesive before use, as utilised, for example, in the manufacture of the panty fastening adhesive patch of commercial sanitary napkins, or pieces of double sided adhesive tapes, etc.

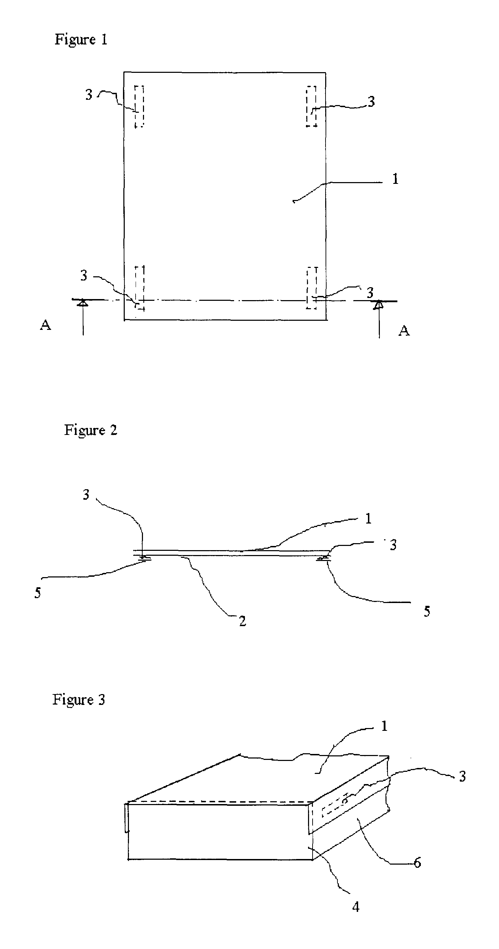

As shown in figure 3, the mattress cover has suitable dimensions to completely cover at least the top surface and partially the longitudinal sides 6 of the mattress 4, while adhesive means 3 are preferably attached to the longitudinal sides 6 of the mattress 4.

Any other suitable shape or arrangement for a mattress cover according to the present invention is also possible, depending on the particular mattress type, shape and dimensions, and on the intended use of the mattress cover.

Preferably the disposable, moisture vapour permeable, liquid impermeable covering sheet of the present invention, when used as a mattress cover or a pillow cover, has a surface area ranging from 15.0 m² to 0.3 m², preferably from 8.0 m² to 0.5 m². Most preferably, particularly in the case of a mattress cover, said surface area is from 5.0 m² to 1.0 m²_{.}

Suitable thermoplastic compositions for the backsheet 2 can be those disclosed in the European patent applications EP 963760 and EP 964026, already mentioned in the background of the invention, but other compositions which form monolithic breathable films can be utilised.

Residual tackiness of the thermoplastic composition can be advantageously provided by the selection of the tackifying resin; indeed the thermoplastic compositions described in the above applications typically include resins or blends of tackifying resins. Preferred resins, which may be present by up to 50% by weight of the thermoplastic composition, may be selected from rosins and rosin esters, hydrocarbon resins, aliphatic resins, terpene and terpene-phenolic resins, aromatic resins, synthetic C5 resins, mixtures of synthetic C5-C9 resins, and mixtures thereof.

By suitably selecting the tackifier resin or blend of tackifier resins, it is possible to adjust the residual tackiness at room temperature of the thermoplastic composition, and therefore of the layer formed from said thermoplastic composition, to the extent that it has the typical characteristics of a pressure sensitive adhesive.

Preferred tackifier resins, or blend of tackifier resins still have a softening point of 125°C or less, and are selected from the group consisting of rosin and rosin esters, terpene-Phenolic resins, aromatic resins, and mixtures thereof.

More preferably, the thermoplastic compositions according to the above mentioned applications comprise a blend of tackifier resins, selected as described above, wherein moreover from 0% to 20%, preferably from 2% to 15%, by weight of said blend of tackifier resins comprises a resin or blend of resins having a softening point of less than 25°C, and from 80% to 100%, preferably from 85% to 98%, by weight of said blend of tackifier resins comprises a resin or blend of resins having a softening point of at least 70°C.

Other thermoplastic compositions for the backsheet 2 are those described in our European patent application No. 00116284.1, filed on 10 August 2000 with title: "Thermoplastic hydrophilic polymeric compositions with improved adhesive properties for moisture vapour permeable structures" (P&G Case CM2406F).

Monolithic films are preferred materials because they do not allow the flow of moisture vapour through open pores or apertures in the material, but do transfer substantial amounts of moisture vapour through the film by absorbing water on one side of the film where the moisture vapour concentration is higher, and desorbing or evaporating it on the opposite side of the film where the moisture vapour concentration is lower. Consequently, while they allow the passage of moisture vapour, they are truly impermeable to liquids, dust, etc.

### Odour control means

According to the present invention the disposable covering sheet for bedding items comprises as an essential component an odour control means.

It is known that in use, articles, which come in direct or indirect contact with human skin tend to acquire a variety of compounds, for example volatile fatty acids (e.g. isovaleric acid), ammonia, amines (e.g. triethylamine), fatty acids, sulphur containing compounds (e.g. mercaptans, sulphides), alcohols, ketones and aldehydes (e.g. furaldehyde) which release unpleasant odours.

These compounds may be present in the bodily fluid or may be produced after by fermentation.

A number of compounds, mixtures, compositions and the like are known in the art to combat some of the unpleasant odours referred to above many of which are based on adsorbents such as activated carbon, clay and zeolites. Particularly, odour control systems are known in the field of the art relating to articles for absorbing bodily fluids such as sanitary napkins, pantiliners, disposable diapers, incontinence pads, tampons, underarm pads and the like.

Odour control means to be used in the covering sheets of the present invention can be selected from the group consisting of odour control materials, as mentioned below. These materials can be typically classified according to the type of odour the material is intended to combat. Odour can be chemically classified as being acidic, basic or neutral.

Acidic odour controlling materials have a pH greater than 7 and typically include sodium carbonates, sodium bicarbonates, sodium phosphates, particularly zinc and copper sulphates.

Basic odour controlling agents have a pH of less than 7 and include compounds such as carboxylic acids such as citric acid, lactic acid, boric acid, adipic acid and maleic acid.

Neutral odour controlling materials have a pH of approximately 7. Examples of these types of compounds include activated carbons, clays, zeolites, silicas, absorbent gelling materials and starches. Neutral odour control materials and systems are disclosed for example in EP 0348978, EP 0510619, WO 91/12029, WO 91/11977, WO 91/12030, WO 81/01643 and WO 96/06589. Also cyclodextrin and derivatives can be used as described in US 5429628. Zeolites and cyclodextrins are particularly preferred as odour control means in the covering sheets of the present invention.

Alternatively, the odour control systems can be categorised with respect to the mechanism by which the malodour detection is reduced or prevented. The above odour control agents typically control odour detection by an adsorptive mechanism.

Therefore, odour control systems which chemically react with malodorous compounds or with compounds which produce malodorous degradation products thereby generating compounds lacking odour or having an odour acceptable to consumers can also be utilised herein. Useful agents include chelating agents selected from amino carboxylates such as for example ethylenediaminetetracetate, as described for example in US 4356190, amino phosphonates such as ethylenediaminetetrakis (methylene-phosphonates), polyfunctionally-substitutes aromatic chelating agents as described in US 3812044 and mixture thereof. It is believed that the benefit of these compounds is in part due to their exceptional ability to remove iron, copper, calcium , magnesium and manganese ions present in the bodily fluid and their degradation products by formation of chelates.

Another useful odour control system for use herein comprises a buffer system, such as citric acid and sodium bicarbonate, sodium phosphate and sorbic acid buffer systems. Also buffer systems having a pH from 7 to 10 as described for example in WO 94/25077 can be used herein.

An alternative odour control system utilises ion exchange resins as those described in US 4289513 and US 3340875.

Also so called anti perspirant such aluminium salts for example aluminium chloridrate and aluminium sulphate and antimicrobics such as Triclosan and benzoic, propionic and sorbic acids for example can also be used as odour control agents. Such agents are described in "The Chemistry and Manufacture of Cosmetics", by W. H. Mueller and R. P. Quatrale and "The Journal of Investigative Dermatology, Vol. 88, N. 3, March Suppl. 1987, entitled "Skin Microflora", by J. J. Leydon, K. D. McGinley et al.

Other useful odour control agents are enzyme blocking agents as described in Cosm. And Toil. 95, 48, 1980, in "Non microbiological deudourising agents" by R. Osberghaus such as triethyl cytrate and odour adsorbers for example zinc ricinoleate as described in Cosmesi Funzionale, pages 465-498, ED. Singerga, 1988, by G. Proserpio.

Further useful art can be find in the following patent publications:

| **Publication number** | **Title** |
|---|---|
| EP-0515477 | High capacity odor controlling compositions |
| EP-0515503 | Carbon containing odor controlling compositions |
| EP-0515473 | Odor controlling compositions and articles |
| EP-0691856 | Odor controlling absorbent articles containing small particle size uncomplexed cyclodextrin and fluid absorbing material |
| EP-0691857 | Odor controlling absorbent articles which provide a positive scent signal through cyclodextrin/perfume inclusion complexes and /or solid cellular matrix perfume |
| EP-0749295 | Catamenials containing an antibacterial agent in an apertured topsheet for odor control |
| EP-959852 | Sanitary napkin with perfume-bearing microcapsule adhesive |
| WO98/10861 | Modified Porous starch |
| WO98/26808 | Absorbent articles with odor control system |
| EP-695197 | Odour control material |
| US4385632 | Gemicidal absorbent body |
| WO96/06589 | Bentonite as odors control material |
| EP-0811387 | Feminine hygiene absorbent products having a zeolite and silica odor control system |
| WO97/46192 | Absorbent articles having an odor control system comprising silica, zeolite and AGM |
| WO97/46191 | Absorbent article having an odor control system comprising a chelating agent and an odor absorbing agent |
| EP-0811391 | Absorbent article having a odor control system comprising a chelating agent |
| W097/46187 | Absorbent article comprising a polyfunctionally substituted aromatic chelating agent for odor control |
| W097/46188 | Absorbent article having an odor control system of zeolite and silica in close physical proximity |
| EP-0811388 | Activated carbon-free absorbent articles having a silica and zeolite odor control system |
| EP-0813848 | Breathable absorbent articles having odor control |
| EP-0811392 | Breathable absorbent article having a chelating agent-based odor-control system |
| EP-0811389 | Breathable absorbent articles having a silica-based odor control system |
| EP-0891708 | Anti-microbic agent |
| WO99/30754 | Compacted odor control particles for use in absorbent articles |
| EP-937467 | Doped odor controlling materials |

In addition, other materials can be used as odour control agents selected from the group consisting of antimicrobics, oxidising agents, lactic acid producing bacillus, chitin, chitosan.

The odour control agent of the present invention is preferably selected from the above agents or combinations thereof, wherein zeolites and cyclodextrin are preferred, but it should be noted that other known odour control agents or systems can be used.

The odour control system can be incorporated in the article of the present invention by any of the methods disclosed in the art.

### Active agents releasing system

According to the present invention the disposable covering sheet can also optionally comprise an active agents releasing system in addition to the odour control means.

Active materials such as perfumes, which will release a fragrance during their utilisation, can be utilised.

As used herein the term perfume means any odoriferous material. In general, such materials are characterised by a vapour pressure less than atmospheric pressure at ambient temperatures. The perfume employed herein will most often be liquid at ambient temperatures, but also can be solids such as the various camphoraceous perfumes known in the art. A wide variety of chemicals are known for perfumery uses, including materials such as aldehydes, ketones, esters and the like. More commonly, naturally occurring plant and animal oils and exudates comprising complex mixtures of various chemical components are known for use as perfumes, and such materials can be used herein. The perfumes herein can berelatively simple in their composition or can comprise highly sophisticated, complex mixtures of natural and synthetic chemical components, all chosen to provide any desired odour.

Typical perfumes herein can comprise, for example, woody/ earthy bases containing exotic materials such as sandalwood oil, civet, patchouli oil and the like.

The perfumes herein can be of a light, floral fragrance, e.g., rose extract, violet extract and the like. The perfumes herein can be formulated to provide desirable fruity odours, e.g., lime, lemon, orange and the like.

Perfume materials are described more fully in S. Arctander, Perfume Flavors and Chemicals. Vols. I and II. Aurthor, Montclair, N.J., and the Merck Index, 8th Edition, Merck & Co., Inc. Rahway, N.J..

In short, any chemically compatible material which exudes a pleasant or otherwise desirable odour can be used in the perfumed particles herein to provide a desirable odour when applied to articles according to the present invention.

Perfumes which are normally solid can also be employed in the present invention. These may be admixed with a liquefying agent such as a solvent prior to incorporation into the articles of the present invention by spraying, or may be simply melted and incorporated in the film layer, as long as the perfume does not sublime or decompose upon heating.

In addition, starch encapsulated perfumes, as described for example in GB 1464616, can be preferably utilised. Encapsulation allows a long lasting release of perfumes.

Useful preferred active agents are those disclosed in WO 00/02987, EP 0971021, EP 0971024, and EP 0971025 which describe, inter alias, a product of reaction between a primary amine and a perfume component. By utilising the product of reaction described by the above patent applications, the release of the active (for example, a perfume component) lasts for a longer period of time than by the use of the active alone itself.

It should be noted that active agents are not limited to perfumes but other know active agents selected from insecticides, deodorants, volatile therapeutic materials, aromatic oils and mixtures thereof can be utilised for providing a benefit to the user.

Volatile therapeutic materials include materials which assist in keeping nasal passages clear of congestion in cold or allergy sufferers, for example menthol, camphor, eucalyptus oil and similar oil, as well as other materials which can benefit a patient by absorption through the nasal passages and the lungs as a result of prolonged exposure. The therapeutic materials may include extracts of herbs and the like which help the user to relax or to sleep. Aromatic oils, such as those referred to as 'aromatherapy oils' may also be used into the substrate.

The active agent, if present, is preferably selected from any of the above agents or combinations thereof.

The active agent or agents can be incorporated in the article of the present invention by any of the methods disclosed in the art.

### Other characteristics

A moisture vapour permeable, liquid impervious layer can be formed from a suitable thermoplastic composition by coating said thermoplastic composition onto a substrate.

A process for making a film layer from a thermoplastic composition typically comprises the steps of providing said composition, heating it to make it flowable, and coating said composition in the molten state onto a substrate in a layer having the desired thickness. It should be noted that odour control materials and active agents can be included in the thermoplastic composition. Alternatively, they can be applied onto the surface of the film or layer made of the thermoplastic composition, e.g. directly adhered thereto by taking advantage of the tackiness of the composition immediately or shortly after formation of the layer or film as mentioned above.

In accordance with the present invention, a moisture vapour permeable, water impervious composite is formed which comprises the thermoplastic composition and a substrate onto which said thermoplastic composition is coated, wherein the substrate is also moisture vapour permeable.

Such embodiment provides a moisture vapour permeable, liquid impervious composite structure wherein the film layer formed from the thermoplastic composition is firmly bonded to the substrate while providing the required performance for liquid barrier and hence could be advantageously provided as thinly as possible. In this manner such embodiment provides a moisture vapour permeable, liquid impervious composite structure wherein the contribution of the film layer formed from the thermoplastic composition to the performance of the composite material resides mainly in the provision of a liquid barrier. The remaining performance physical criterion being preferably provided by the provided substrate, that therefore preferably acts also as a support layer. However it should be noted that the film layer does not need to be as thin as possible in any application because the thermoplastic compositions, according to our before mentioned patent applications, provide high breathability (high WVTR) also in the case of thicker film layers. Typical thickness of the film layer of the thermoplastic composition applied onto a suitable substrate to form a composite structure according to the present invention range from 2 µm to 200 µm.

The substrate, or support layer may be any useful layer which is also moisture vapour permeable, preferably having a moisture vapour permeability of at least 400 g/m²•24h, more preferably at least 500 g/m²•24h, and most preferably at least 600 g/m²•24h.

Preferred support layers for use herein include woven and nonwoven layers, most preferably hydrophobic fibrous layers such as hydrophobic nonwoven. As for non limiting examples, a SMS (Spunbonded-Meltblown-Spunbonded) hydrophobic 100% polypropylene nonwoven of 17 g/m² or of 25 g/m² both commercialised under the code G17Al0, MD3000, respectively, by Corovin - BBA Nonwovens Group (Germany), or a spunlaced nonwoven of 50 g/m² commercialised under the code TJET C500 by Tecnofibra (Italy) function well.

Suitable substrates for use herein as further layers in the structure according to the present invention could also include two dimensional, planar micro and macro-porous films; macroscopically expanded films; formed apertured films; nonwoven and woven layers. Suitable macroscopically expanded films for use herein include films as described in for example in US 4,637,819 and US 4,591,523.

The structures of the preferred embodiment according to the present invention are particularly advantageous as they allow the possibility of providing a composite wherein the thermoplastic composition may be applied onto the support substrate as a film layer with the desired thickness. Typical coating conditions and apparatuses known in the art for the direct coating of low viscosities hot melts can be readily utilised in order to provide the thermoplastic composition at the desired thickness.

A possible method for forming a composite laminate by coating the thermoplastic composition onto a substrate acting as a support layer is described in PCT application WO 96/25902.

At least at the coating temperature, the thermoplastic composition in form of a layer preferably exhibits adhesive properties on the supportive substrate in order to form the preferred composite such that no additional adhesive is required to achieve a permanent attachment between the thermoplastic composition and the substrate.

A moisture vapour permeable, liquid impervious composite structure formed by coating a thermoplastic hydrophilic composition onto a suitable substrate finds particular utility as a structure constituting a disposable, moisture vapour permeable, liquid impermeable mattress cover according to the present invention.

### Example 1

A polyether-amide block copolymer available from Atofina (France) commercialised under the trade name Pebax 2533 SN is compounded with Tri Butyl Citrate (plasticiser) available from Aldrich Co., Res A-2691 (tackifier resin) available from Hercules Inc., Res A-2690 (tackifier resin) available from Hercules Inc., both resins described in our already mentioned European application No. 00116284.1, and Irganox 1010 (anti oxidant agent) available from Ciba-Geigy.

The final formulation in percent by weight has the following composition:

| | |
|---|---|
| 44% | Pebax 2533 SN |
| 25% | Tri Butyl Citrate |
| 15% | Res A-2691 |
| 15% | Res A-2690 |
| 1% | Irganox 1010 |

The blend is melt extruded to obtain a film having a basis weight of 20 g/m². The film is laminated directly onto a substrate constituted by a SMS (Spunbonded-Meltblown-Spunbonded structure) hydrophobic 100% polypropylene nonwoven 25 g/m² (support layer) commercialised under the trade name MD3000, available form Corovin - BBA Nonwovens Group (Germany). The composite has a WVTR (Water Vapour Transmission Rate) of 846 g/m²•24h.

The composite structure is used to produce a mattress cover, e.g. a bed mattress cover as illustrated in the herein drawings. It has a flat rectangular shape with suitable dimensions to completely cover at least the upper surface of a bed mattress, wherein the film layer 2 of thermoplastic composition is intended to face, in use, the mattress 4, and to directly contact it. In particular, the dimension are: 1.75 m (width) and 2.10 m (length) to completely cover the upper surface of a bed mattress 4 and partially its longitudinal sides 6 as indicated in figure 3. The corners of the mattress cover are fixed to the corners of the bed mattress by adhesive tapes 3, after detaching the release liners 5 which protect the adhesive surface of adhesive tapes before use. The adhesive tapes 3 are pieces of double sided adhesive tape, each having a width of 50 mm and a length of 300 mm, available from Avery Dennison Co. under FT310 code.

It should be noted that other adhesive tapes could be provided along the longitudinal edges of the mattress cover for better fixing it to the longitudinal sides 6 of a bed mattress 4.

Samples of the above material for mattress cover having 10 cm x 10 cm dimensions were sprayed with different amounts of a water solution of cyclodextrin at 2% by weight to obtain two different amounts of cyclodextrin in the samples, respectively 8 mg/sample (0.8 g/m²) and 16 mg/ sample (1.6 g/m²). The cyclodextrin was a beta cyclodextrin available under the code 28707 from Fluka Div. of Sigma-Aldrich Co.

### Odour Control Test

An amount of 0.5 ml of malodorant solution was added to a cup whose opening, having a diameter of 50 mm, was covered and suitably sealed with the treated samples (i.e. samples containing the odour control material) and with a sample having the same structure, but without odour control material.

The composition ( by weight) of the malodorant solution or mixture was:

| | |
|---|---|
| distilled water | 99.000% |
| sodium chloride | 0.700% |
| butyric acid | 0.040% |
| ascorbic acid | 0.040% |
| lactic acid | 0.100% |
| formic acid | 0.009% |
| propionic acid | 0.006% |
| capronic acid | 0.004% |
| urea | 0.101% |

The cups with the samples were put into an oven at 30 °C and 75% relative humidity. After two hours a panel of trained odor graders assessed malodour intensity over the samples on a scale from 0 to 10. In particular intensity scale was:

| | |
|---|---|
| 10 | high |
| 9 | high |
| 8 | high |
| 7 | high |
| 6 | medium |
| 5 | medium |
| 4 | medium |
| 3 | low |
| 2 | low |
| 1 | low |
| 0 | No odour |

Each expert grader gave the number of odour intensity after having smelled the treated and untreated samples, and an average value was obtained.

The results are reported in the following table as percentage of malodour (odour intensity) versus the reference which was the same as the described samples but not treated with the cyclodextrin solution. The average of the results of the tests performed are reported, wherein the odour intensities are expressed as percentages related to the odour intensity of the reference, which has been considered to correspond to 100%.

| Sample | Odour intensity % |
|---|---|
| Reference | 100 |
| Sample with 0.8 g/ m2 of cyclodextrin | 95 |
| Sample with 1.6 g/ m2 of cyclodextrin | 75 |

As it is evident from the above data adding 1.6 g/m² of cyclodextrin was able to reduce the odour intensity by 25% which can give a significant benefit to the user in terms of malodour reduction, while adding 0.8 g/m² of cyclodextrin, which reduced the odour intensity by 5%, cannot give the same benefit. As a matter of fact, it was surprisingly discovered that an odour control system to be effective and , consequently, to be able of giving a consumer benefit in terms of malodour reduction, should reduce the odor intensity (malodour reduction) of at least 15%.

### Example 2

As example 1 except that this time zeolite ZP-4A available from Silkem d.o.o. (Slovenija) was added into the thermoplastic blend composition in proportion of 1 % by weight of composition as odour control material.

When evaluated for malodour reduction in the same manner as in the example 1 the sample of mattress cover comprising the composition containing 1% of zeolite gave a malodour reduction of 29% compared to the sample of mattress cover of the same composition but not containing zeolite.

Further increase of the amount of zeolite gave no significant further advantage. In fact at a zeolite level of 2% the malodour reduction was 30%.

A decrease of zeolite level at 0.5% gave no advantage. In fact at a zeolite level of 0.5% the malodour reduction was 5% only.

### Example 3

A polyether-amide block copolymer available from Atofina (France) commercialised under the trade name Pebax 2533 SN01 is compounded with Citroflex 2 (plasticiser) available from Reilly Chemical Co., Ketjenflex 8 (plasticizer) available from Akzo Nobel Co., Irganox 1010 and Irganox PS 800 (both anti oxidant agents) available from Ciba-Geigy, and ZP-4 A (zeolite - as odour control material) available from Silkem d.o.o. (Slovenija).

The final formulation in percent by weight has the following composition:

| | |
|---|---|
| 50.00% | Pebax 2533 SN |
| 24.35% | Citroflex 2 |
| 24.35% | Ketjenflex 8 |
| 0.15% | Irganox 1010 |
| 0.15% | Irganox PS 800 |
| 1.00% | ZP-4 A |

The blend is melt extruded to obtain a film having a basis weight of 20 g/m². The film is laminated directly onto a substrate constituted by a SMS (Spunbonded-Meltblown-Spunbonded structure) hydrophobic 100% polypropylene nonwoven 17 g/m² (support layer) commercialised under the trade name G17Al0, available form Corovin - BBA Nonwovens Group (Germany). The composite has a WVTR of 1907 g/m²•24h. Then a conventional starch encapsulated perfume (50% active agent and 50% starch) having fresh notes was mixed with talcum and the mixture was applied at a level of 3.80 g/m² (with a starch encapsulated perfume at a level of 0.3 g/m²) and fixed onto the film by the adhesiveness (i.e. residual tackiness) of the film itself.

The composite structure is used to produce a mattress cover, e.g. a bed mattress cover as previously described in the example 1.

The above disposable, moisture vapour permeable, liquid impervious mattress cover demonstrated in confidential in-use tests to be able to decrease malodours while, at the same time, releasing perfume and smelling fresh for many days.

### Tests.

Moisture vapour permeability is measured as Water Vapour Transmission Rate (WVTR) at 23°C and 50% relative humidity according to the modified ASTM E-96 "Upright Cup" method. The only modification to the standard ASTM E-96 "Upright Cup" method consists in a change in the height of the air gap between the sample and the water surface in the cup, which height is 3 mm ± 0.5 mm, instead of 19 mm ± 2.5 mm, as specified in the standard test method.

The Odour Control Test is illustrated in the context of the example 1.

## Claims

1. A disposable, moisture vapour permeable, liquid impermeable, covering sheet comprising at least a moisture vapour permeable layer and a liquid impermeable but moisture vapour permeable film layer of thermoplastic composition applied onto said moisture vapour permeable layer **characterised in that** said covering sheet comprises odour control means which are able to provide a malodour reduction of at least 15%, as measured according to the Odour Control Test described herein.

2. A disposable, moisture vapour permeable, liquid impermeable covering sheet according to claim 1 wherein said odour control means comprises cyclodextrin and/or zeolite.

3. A disposable, moisture vapour permeable, liquid impermeable, covering sheet according to any preceding claim further **characterised in that** said covering sheet comprises an active agent releasing system.

4. A disposable, moisture vapour permeable, liquid impermeable, covering sheetaccording to claim 3 **characterised in that** said active agent releasing system is able to provide a long lasting release of active agents.

5. A disposable, moisture vapour permeable, liquid impermeable covering sheet according to claim 3 or claim 4 wherein said active agent is a perfume.

6. A disposable, moisture vapour permeable, liquid impermeable covering sheet according to claim 3 or claim 4 wherein said active agent is a perfume and said odour control means is a zeolite.

7. A disposable, moisture vapour permeable, liquid impermeable covering sheet according to claim 5 or 6 wherein said perfume is a starch encapsulated perfume.

8. A disposable, moisture vapour permeable, liquid impermeable, covering sheet according to any preceding claims **characterised in that** its Water Vapour Transmission Rate (WVTR) is at least 400 g/m²•24h, preferably at least 500 g/m²•24h, and more preferably at least 600 g/m²•24h.

9. A disposable, moisture vapour permeable, liquid impermeable, covering sheet according to any preceding claim wherein said covering sheet is a disposable covering sheet for bedding articles.

10. A disposable covering sheet for bedding articles according to claim 9 wherein said covering sheet is a disposable mattress cover or a disposable pillow cover.

11. A disposable mattress cover according to claim 10 **characterised in that** said mattress cover has at least four adhesive means 3 for fixing it to the longitudinal sides 6 of a bed mattress 4.
